# EUROPEAN PATENT APPLICATION

(11) **EP 2 100 602 A1**
(43) Date of publication of application: **16.09.2009**
(21) Application number: 08152647.7
(22) Date of filing: 12.03.2008
(51) Int. Cl.: A61K 31/167, A61K 31/381, A61K 31/415, A61K 31/4155, A61K 31/42, A61K 31/425, A61K 31/445, A61P 17/02, A61P 31/04

(54) **Method and compositions suitable for treatment of wounds**

(71) Applicant: QuoNova Europe GmbH, 82152 München (DE)
(72) Inventor: The designation of the inventor has not yet been filed
(74) Representative: Vossius & Partner

(57) **Abstract**

Compositions for the treatment of areas of compromised skin integrity as well as pharmaceutical preparations and medicaments containing them are provided. Methods of treatment and prevention of microbial damages and diseases, in particular for diseases of compromised skin integrity where there is an advantage in inhibiting quorum sensing regulated phenotypes of pathogens is also provided.

## Description

### BACKGROUND OF THE INVENTION

### Technical Field of the Invention

The present invention relates to compositions containing compounds capable of controlling bacterial pathogens. In particular, the invention relates to compositions for reducing or inhibiting bacterial colonization and infection in areas of compromised skin integrity, as well as methods for the treating the same.

### Description of Related Art

Many microorganisms, including bacteria, fungi, protozoa and algae cause severe damages or diseases in different areas such as industry, agriculture, environment and medicine. Especially bacteria, as human pathogens, cause tremendous costs in public health systems worldwide. The continuing emergence of multiple-drug-resistant bacterial strains has necessitated finding new compounds that can be used in antibacterial treatment. There are two broad strategies for the control of bacterial infection: either to kill the organism or to attenuate its virulence such that it fails to adapt to the host environment. The latter approach has, however, lacked specific targets for rational drug design. The discovery that bacteria employ signal transduction pathways comprising small molecules or peptides to globally regulate the production of virulence determinants offers such novel targets.

A wide variety of Gram-negative bacteria produce *N*-acyl-L-homoserine lactone (AHL or HSL) derivatives as signal molecules in intercellular communication. These molecules, also referred to as "pheromones" or "quoromones", comprise a homoserine lactone moiety linked to an acyl side chain. Bacteria use this signalling system to monitor their population cell density in a process referred to as "quorum sensing." In each cell of a population, an HSL synthase from usually the LuxI family of proteins produce a low basal level of diffusible HSLs. The HSL concentration increases with bacterial population density until a threshold concentration is reached which results in expression of various HSL-dependent genes through an HSL-receptor protein belonging generally to the LuxR family of transcriptional regulators. This HSL-receptor protein complex serves not only as positive transcription regulator of quorum sensing regulated genes but also as positive regulator for the HSL synthesis itself. Therefore, the entire system is amplified via a process of autoinduction.

This system was first discovered in the bioluminescent marine bacteria *Vibrio harveyi* and *V. fischeri* where it is used to control bioluminescence expression. In recent years it has become apparent that many Gram-negative bacteria employ one or more quorum sensing systems comprising HSL derivatives with different acyl side chains to regulate in a cell-density dependent manner a wide variety of physiological processes such as swarming motility, biofilm formation, pathogenicity, conjugation, bioluminescence or production of pigments and antibiotics (See, e.g.: Fuqua et al., Ann. Rev. Microbiol. 50:727-51, 1996; Fuqua & Greenberg, Curr. Opinion Microbiol. 1:183-89, 1998; Eberl, Syst. Appl. Microbiol. 22:493-506, 1999; De Kievit & Iglewski, Infect. Immun. 68:4839-49, 2000).

With regard to bacteria that utilize HSL-based quorum sensing as part of their lifestyle, *Pseudomonas aeruginosa* is perhaps the best understood in terms of the role quorum sensing plays in pathogenicity. In this human opportunistic pathogen which has an extremely high potential to develop resistance mechanisms against traditional antibiotic treatment, production of many virulence factors, components of the protein secretion apparatus, efflux transporters, production of alginate and adhesins, twitching motility and pilin export is regulated by two interlinked quorum sensing circuits. Moreover, it has been demonstrated that this signaling system is involved in the ability of *P. aeruginosa* to form biofilms (Davies et al., Science 280:295-8, 1998). Huber *et al*. has demonstrated that biofilm formation and swarming motility of *Burkholderia cepacia*, like *P. aeruginosa* a human opportunistic pathogen, is also dependent on an HSL-based quorum sensing system (See Microbiology 147:2517-28, 2001).

Gram-positive bacteria like staphylococci have developed different quorum-sensing systems that enable cell-to-cell communication and regulation of numerous colonization and virulence factors. The staphylococcal accessory gene regulator (*agr*) quorum-sensing system decreases the expression of several cell surface proteins and increases the expression of many secreted virulence factors in the transition from late-exponential growth to stationary phase in vitro. Expression of *agr* was found to contribute to staphylococcal pathogenesis in several infection models, including murine subcutaneous abscesses and arthritis, as well as rabbit endocarditis. Expression of *agr* also appears to be involved in the invasion and apoptosis of epithelial cells. Two primary transcripts, RNAII and RNAIII, are generated by the *agr* locus and originate from the P2 and P3 promoters, respectively. The P2 operon encodes four proteins that generate the *agr*-sensing mechanism. AgrB is a transmembrane protein that appears to be involved in (a) processing of the *agrD* product into an octapeptide; (b) secretion of the autoinducing peptide (AIP) signal; and (c) modification of the AIP by the formation of a cyclic thiolactone bond between an internal cysteine and the carboxyl terminus. AgrA and AgrC form a two-component regulatory system in which the transmembrane component, AgrC (histidine kinase), binds the extracellular AIP and in turn modulates the activity of AgrA, the response regulator. Through an as-yet-undefined mechanism, AgrA activity then leads to greatly increased P2 and P3 transcription in the late-log phase of growth, when the concentration of the signal in the medium is high. Sequence variation in *agrB, agrD*, and *agrC* has led to the identification of at least four *S. aureus agr* specificity groups in which AIP produced by one group inhibits *agr* expression in other groups (for a review see: Yarwood and Schlievert, J. Clin. Invest. 112: 1620-1625, 2003).

*Staphylococcus aureus* and *Staphylococcus epidermidis* normally colonize the epithelial surfaces of large numbers of humans. *S. epidermidis* is considered part of the normal human microbial flora, while *S. aureus* is usually regarded as a transient member. Colonization by either species usually does not lead to adverse events. However, when these organisms or their extracellular products are allowed to breach the epithelial layer, serious disease can result. *S. aureus* has many cell surface virulence factors (such as protein A and clumping factor) and secreted exotoxins and enzymes that allow strains to cause a myriad of infections. These diseases range from relatively benign furuncles and subcutaneous abscesses to scalded skin syndrome, sepsis, necrotizing pneumonia, and toxic shock syndrome (TSS). While no single cell surface virulence factor has been shown to be uniquely required for mucous membrane attachment, once colonization occurs, numerous secreted exotoxins, including the pyrogenic toxin superantigens and exfoliative toxins, definitively cause serious human disease. Other secreted exotoxins, such as the four hemolysins (α, β, δ, and γ) and Panton-Valentine leukocidin have also been suggested to contribute to significant illnesses. S. *epidermidis* does not possess the array of extracellular toxins that *S. aureus* does, and its primary virulence factor is considered to be its ability to form biofilms.

Biofilms are defined as an association of microorganisms growing attached to a surface and producing a slime layer of extracellular polymers in which the microbial consortia is embedded in a protective environment (for a review see: Costerton et al., Ann. Rev. Microbiol. 49:711-65, 1995). Biofilms represent a severe problem as bacteria integrated in such a polymer matrix develop resistance to conventional antimicrobial agents. *P. aeruginosa* cells, for example, growing in an alginate slime matrix have been demonstrated to be resistant to antibiotics (e.g., aminoglycosides, β-lactam antibiotics, fluoroquinolones) and disinfectants (Govan & Deretic, Microbiol. Rev. 60:539-74, 1996). Several mechanisms for biofilm-mediated resistance development have been proposed (Costerton et al., Science 284:1318-22, 1999).

One solution to the problem of proliferation of biofilm formation is through treatment with compounds that block quorum sensing processes. U.S. Patent Application Publication No. 2004/0235914 and U.S. Patent Application Publication No. 2004/0063765 disclose compounds that selective modulate bacterial cell-cell communication. Through inhibition of this communication system, the expression of many HSL-dependent virulence genes and other phenotypes like swarming motility and biofilm formation are significantly reduced or completely abolished. While these compounds have proven effective in a variety of applications, the treatment of microorganisms, such as bacteria, that are found in or around an area of compromised skin integrity present a unique problem. That is, if left untreated, bacteria may colonize the region which can lead to symptoms of infection which include erythema, warmth, swelling, pain, and odor. Colonization occurs when the bacteria replicate and adhere to the wound surface, but do not cause damage to the host. Wound infection is characterized by the presence of replicating bacteria within a wound resulting in a subsequent host response. In some cases, systemic infection with fever may result from infection of areas of compromised skin integrity, causing elevated white blood cell count and sepsis. Necrotic tissue may also develop in the wound bed if left untreated.

Areas of compromised skin integrity may become colonized by a variety of bacteria. *Pseudomonas aeruginosa*, for example, is a frequent cause of nosocomial infections such as infections in areas where the integrity of a physical barrier to infection such as the skin is lost through a wound or burn. Secondary wound infections caused by bacteria such as *Pseudomonas aeruginosa* often occur in patients with decubiti, eczema, and tenia pedis. *Pseudomonas aeruginosa* is also a common cause of hot tub or swimming pool folliculitis which can cause pruritic follicular, maculopapular, vesicular, or pustular lesions on any part of the body that was immersed in water. *Pseudomonas* also has emerged as an important source of burn wound sepsis.

Bacterial infections are especially prominent in chronic wound patients. Many chronic wound patients may possess impaired host defense mechanisms which, in turn, lower the body's ability to respond to invasion by pathogens such as *Staphylococcus*, *Streptococcus* and *Pseudomonas aeruginosa*. The ability of pathogens in the wound bed to create a damaging effect on the host is dependent on the relative number of organisms, their virulence and whether or not they invade the tissue.

Various antibacterial and prophylactic treatments are currently used to treat areas of compromised skin. Ionic silver formulations can provide broad spectrum antimicrobial coverage against yeast, fungi, viruses and Gram-negative and Gram-positive bacteria, including MRSA and VRE commonly found on the skin. Silver also appears to reduce chronic inflammation, which can interfere with wound healing. Dressing applications can provide sustained release of silver, allowing for longer intervals between dressing changes. Formulations of silver sulfadiazine have also gained wide-spread acceptance as an antimicrobial treatment for compromised skin, especially burn wounds and chronic wounds. In high concentrations, silver sulfadiazine has been demonstrated as one of the few antimicrobial compounds effective against both planktonic bacteria as well as fully developed *in vitro* biofilms of *Pseudomonas aeruginosa* (Bjarnsholt et al., APMIS. 115: 921-8, 2007). High concentrations of silver ions, however, must contact the compromised skin to impart a therapeutic effect and frequent dressing changes are suggested.

### SUMMARY OF THE INVENTION

In accordance with the invention, compositions for reducing or inhibiting bacterial colonization and infection in areas of compromised skin integrity, as well as methods for the treating the same are provided. The compositions provided not only generally reduce the presence of planktonic bacteria, but also reduce counts of biofilm, thus reducing the biofilm formation of human pathogens such as *Pseudomonas aeruginosa*, especially in and around areas of compromised skin integrity. The compositions comprise, in part, compounds that are capable of inhibiting a HSL-regulated process by inhibiting bacterial signalling.

Thus, the invention provides compounds according to Formula (I)A, Formula (II)A or Formula (III)A as defined further below.

### Furthermore, the invention provides compounds according to Formula (I):

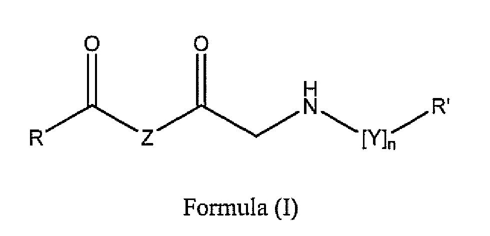

wherein R is an optionally substituted C₁-C₂₀-alkyl group;
Z is N or C;
R' is a 5 or 6-membered monocyclic or polycyclic optionally substituted aromatic or non-aromatic ring system which may contain one or more group(s) X, wherein X is selected from the group consisting of S, O, N, SO or SO₂;
the substituted ring system carries a substituent R^{"} on one or more of the carbon atoms of said ring system;
R" is independently H, OH, S, hydroxyalkyl, hydroxyalkylamine, cycloalkyl, halogen, haloalkyl, haloalkyloxy, NO₂, CN, alkyl, aryl or heteroaryl;
Y is an optionally substituted C₁-C₂₀-alkyl group; n is 0 or 1

In an alternative embodiment, the compositions of the present invention comprise, in part, compounds of Formula (II): 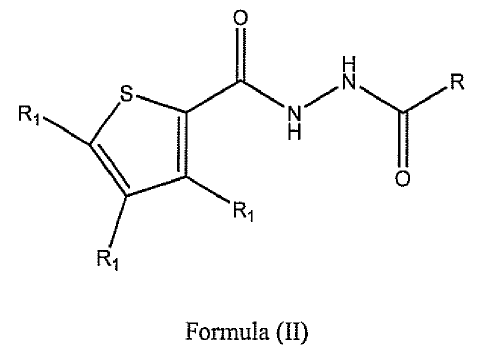 wherein R is a 5 or 6-membered monocyclic or polycyclic optionally substituted aromatic or non-aromatic ring system which may contain one or more group(s) X, wherein X is selected from the group consisting of S, O, N, SO or SO₂; and
wherein R₁ is interpedently of each other H, halogen, NO₂, CN, alkyl, OH, cycloalkyl, SH, alkylthio, hydroxyalkylamino, hydroxyalky, aryl or heteroaryl.

In yet another embodiment, the compositions of the present invention comprise, in part, compounds of Formula (III): 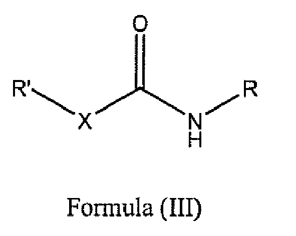 wherein R is a 5 or 6-membered monocyclic or polycyclic optionally substituted aromatic or non-aromatic ring system which may contain one or more group(s) X, wherein X is selected from the group consisting of S, O, N, SO or SO₂; and
X is C or N;
R' is an optionally substituted C₁-C₂₀-alkyl group.

Such quorum sensing blocking compounds include those compounds listed in Table 1. The compositions of this invention generally do not show any toxic effect and are therefore suitable for applications such as new antibiotic therapeutics, pharmaceuticals and medicaments. The present compositions can also comprise a therapeutically effective amount of at least one compound capable of either preventing infection by inhibiting the growth or action of at least one bacterium (e.g., bacteriostat) or capable of destroying organisms that might carry disease (e.g., antimicrobial) or a combination thereof.

In another aspect, the present invention provides a method of treating areas of compromised skin. In yet another aspect, the present invention provides a method for the treatment or prophylaxis of compromised skin where there is an advantage in inhibiting microbial colonization. Both methods comprise the administration of a therapeutically effective amount of a composition containing at least one biofilm inhibiting compound or physiologically acceptable salts or physiologically functional derivatives thereof and a therapeutically effective amount of at least one bacteriostatic or antimicrobial compound or a combination thereof.

### BRIEF DESCRIPTION OF THE DRAWINGS

Figure 1 illustrates the experimental design utilizing the inventive compositions comprising quorum sensing compounds (i.e., no silver sulfadiazine).
Figure 2 illustrates the experimental design utilizing the inventive compositions comprising silver sulfadiazine.

### DETAILED DESCRIPTION OF A PREFERRED EMBOBIDMENT

The term "biofilm" refers to cell aggregations comprising either a single type of organism or a mixture of more than one organism, then also referred to as "mixed biofilms."

The term "physiologically functional derivative" as used herein refers to compounds which are not pharmaceutically active themselves but which are transformed into their pharmaceutical active form *in vivo*, i.e. in the subject to which the compound is administered.

The term "quorum sensing" is intended to describe cell-density dependent gene regulation through a diffusible signal molecule (Fuqua et al., J. Bacteriol. 176:269-75, 1994).

The compounds of Formulas (I)-(III), as well as (I)A - (III)A can be prepared from a variety of methods such as disclosed in U.S. Patent Application Publication No. 2004/0235914 and U.S. Patent Application Publication No. 2004/0063765, each of which are hereby incorporated herein by reference in their entirety.

Subject of the present invention are compounds according to Formula (I)A for the treatment of areas of wounds: 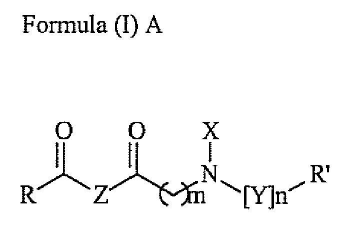 wherein R is a C₁-C₂₀-alkyl group, wherein one or two non-consecutive methylene groups may independently be replaced by O or S, wherein the C₁-C₂₀-alkyl group may optionally be substituted by a group selected from the group consisting of alkyl, cycloalkyl, -OR^{b}, -SR^{b}, - CO₂R^{b}, -CONXR^{b}, -SO₂R^{b}, -NXSO₂R^{b}, -NX₂, fluoro, trifluoroalkyl, optionally substituted hydroxyalkyl, optionally substituted aminoalkyl, optionally substituted aryl, optionally substituted heteroaryl, *N*-methylpiperazinyl, *N*-morpholinyl, wherein R^{b} may independently be selected from H or C₁-C₅ alkyl;
X is H or a C₁-C₃-alkyl group;
Z is NH or CH₂ or NX;
R' is a 5 or 6-membered optionally substituted aromatic or non-aromatic ring system wherein one or more of the carbon atoms may be replaced by a group X^{b}, wherein X^{b} is selected from the group consisting of S, O, N, SO or SO₂;
the substituted ring system carries one or more substituents R" on the carbon or hetero atoms of said ring system;
R" is independently H, OH, SH, O-alkyl, hydroxyalkyl, hydroxyalkylamine, cycloalkyl, halogen, haloalkyl, haloalkyloxy, NO₂, CN, alkyl, aryl or heteroaryl;
Y is an optionally substituted C₁-C₂₀-alkyl group, preferably a C₁-C₁₂-alkyl group, more preferably a C₁-C₆-alkyl group, most preferably a C₁-C₃-alkyl group, wherein said alkyl group may be substituted with alkyl groups, preferably C₁-C₃-alkyl groups. n and m are independently 0 or 1.

Compounds according to formula (I)A are preferred with R' being a optionally substituted pyrrole, pyrazole, imidazole, imidazoline, imidazolidine, oxazole, oxazoline, oxazolidine, isoxazole, isoxazoline, isoxazolidine, thiazole, thiazoline, thiazolindine, isothiazole, isothiazoline, isothiazolidine, thiophen, furan, phenyl, cyclopentadien, hydroxyphenyl, or piperidine.

Compounds according to formula (I)A are especially preferred with R' being a optionally substituted isothiazole, phenyl, pyrazole, cyclopentadiene, hydroxyphenyl, or isoxazole.

Especially preferred are the aforementioned compounds according to formula (I)A in case of R' being a pyrazole wherein the pyrazole is N-substituted, even more especially preferred by C₁-C₄-alkyl. Said pyrazole ring may preferably be substituted by a further group on one of its carbon atoms, more preferably by C₁-C₄-alkyl, even more preferably by a butyl group, most preferably by a t-butyl group.

Especially preferred are the aforementioned compounds according to formula (I)A in case of R' being a isothiazole wherein the isothiazole is substituted by C₁-C₄-alkyl.

Especially preferred are the aforementioned compounds according to formula (I)A in case of R' being a phenyl wherein the phenyl is substituted by F, Cl, Br or CF₃.

Especially preferred are the aforementioned compounds according to formula (I)A in case of R' being a cyclopentadien wherein the cyclopentadien is substituted by C₁-C₄-alkyl.

Further, compounds according to formula (I)A are preferred with R being an optionally substituted C₆-C₁₂-alkyl group, wherein one or two non-consecutive methylene groups may independently be replaced by O or S and the substituents may be as defined above. More preferred in this context are compounds according to formula (I)A with R being an optionally substituted C₇-C₉-alkyl group, most preferred a C₉-alkyl group.

Compounds according to formula (I)A are preferred with m = 0.

In one embodiment, the compositions of the present invention comprise further, compounds of Formula (I): 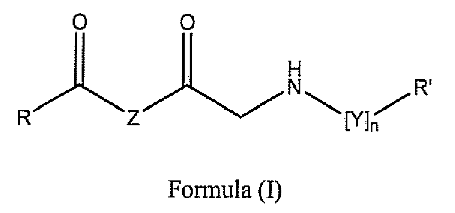 wherein R is an optionally substituted C₁-C₂₀-alkyl group;
Z is N or C;
R' is a 5 or 6-membered monocyclic or polycyclic optionally substituted aromatic or non-aromatic ring system which may contain one or more group(s) X^{b}, wherein X^{b} is selected from the group consisting of S, O, N, SO or SO₂;
the substituted ring system carries a substituent R" on one or more of the carbon atoms of said ring system;
R" is independently H, OH, S, hydroxyalkyl, hydroxyalkylamine, cycloalkyl, halogen, haloalkyl, haloalkyloxy, NO₂, CN, alkyl, aryl or heteroaryl;
Y is an optionally substituted C₁-C₂₀-alkyl group;
n is 0 or 1.

The definitions and preferred embodiments as outlined for formula (I)A apply to Formula I as well.

Thus, in one embodiment subject of the present invention are compounds according to Formula (I) for the treatment of areas of wounds.

In an alternative embodiment subject of the present invention are compounds according to Formula (II)A for the treatment of areas of wounds: 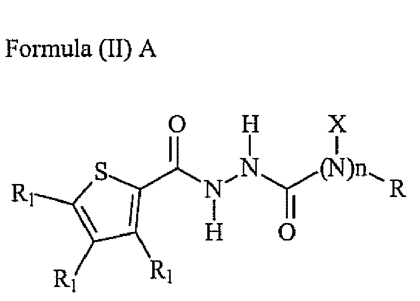 wherein R is an aromatic or non-aromatic ring system consisting of one or two 5- or 6-membered rings wherein in each ring one or more of the carbon atoms may be replaced by a group X^{b}, wherein X^{b} is selected from the group consisting of S, O, N, SO or SO₂, wherein the ring system may be unsubstituted or substituted with one or more residues selected from the group consisting of alkyl, alkyloxy, optionally substituted aryl, halogen, trifluoromethyl, - OCF₃, NX₂, SO₂-alkyl, pyrrolyl, CO₂H, CO₂R^{b}, wherein R^{b} may independently be selected from H or C₁-C₅ alkyl;
wherein R₁ is independently of each other H, halogen, NO₂, CN, alkyl, OH, cycloalkyl, SH, alkylthio, hydroxyalkylamino, hydroxyalky, aryl or heteroaryl,
wherein X is H or a C₁-C₃-alkyl group;
n is 0 or 1.

In the ring systems in the compounds according to the present invention, the replacement of a carbon atom corresponds to a replacement of the carbon atom, including putative hydrogen atoms attached thereto, by the specified hetero atom groups. In ring systems consisting of two rings, the rings may share a certain number of atoms, such as for instance in naphthyl derivatives, which comprise two 6-membered rings sharing two of their atoms.

Compounds according to formula (II)A are preferred with R being an optionally substituted pyrrole, pyrazole, imidazole, imidazoline, imidazolidine, oxazole, oxazoline, oxazolidine, isoxazole, isoxazoline, isoxazolidine, thiazole, thiazoline, thiazolidine, isothiazole, isothiazoline, isothiazolidine, thiophen, furan, phenyl, cyclopentadien, hydroxyphenyl,or piperidine.

Compounds according to formula (II)A are especially preferred with R being an optionally substituted pyrazole, thiophen, or phenyl.

Especially preferred are the aforementioned compounds according to formula (II)A in case of R being a pyrazole wherein the pyrazole is N-substituted, even more especially preferred by C₁-C₄-alkyl. Said pyrazole ring may preferably be substituted by a further group on one of its carbon atoms, more preferably by C₁-C₄-alkyl, even more preferably by a butyl group, most preferably by a t-butyl group.

Especially preferred are the aforementioned compounds according to formula (II)A in case of R being a phenyl wherein the phenyl is substituted by one or more F, Cl, Br or CF₃.

Especially preferred are the aforementioned compounds according to formula (II)A in case of R being a thiophen wherein the thiophen is substituted by one or more F, CI or Br.

Compounds according to formula (I)A are preferred with at least one R₁ being a F, Cl, Br or a C₁-C₃-alkyl group,
In an alternative embodiment, the compositions of the present invention comprise further, compounds of Formula (II): 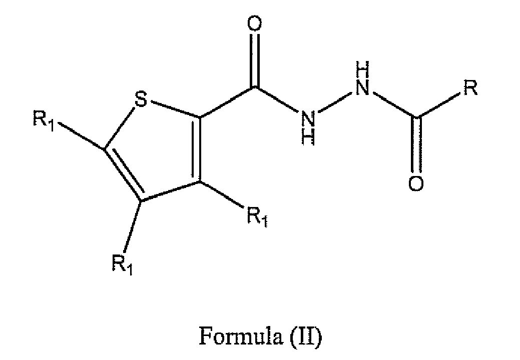 wherein R is a 5 or 6-membered monocyclic or polycyclic optionally substituted aromatic or non-aromatic ring system which may contain one or more group(s) X^{b}, wherein X^{b} is selected from the group consisting of S, O, N, SO or SO₂; and
wherein R₁ is interpedently of each other H, halogen, NO₂, CN, alkyl, OH, cycloalkyl, SH, alkylthio, hydroxyalkylamino, hydroxyalky, aryl or heteroaryl.

The definitions and preferred embodiments as outlined for formula (II)A apply to Formula II as well.

Thus, in one embodiment subject of the present invention are compounds according to Formula (II) for the treatment of areas of wounds.

In an alternative embodiment subject of the present invention are compounds according to Formula (III)A for the treatment of areas of wounds: 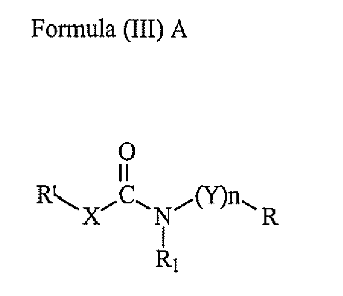 wherein R is an aromatic or non-aromatic ring system consisting of one or two 5- or 6-membered rings wherein in each ring one or more of the carbon atoms may be replaced by a group X^{b}, wherein X^{b} is selected from the group consisting of S, O, N, SO or SO₂, wherein the ring system may optionally be substituted with one or more groups selected from C₁-C₅-alkyl or halogen, more preferably selected from the group consisting of methyl, cyclopropyl, *t*-butyl, F and CF₃;
R₁ is H or a C₁-C₃-alkyl group, or
R₁ and R taken together may form a 5 or 6-membered optionally substituted aromatic or non-aromatic ring system wherein one or more of the carbon atoms may be replaced by a group X^{b}, wherein X^{b} is selected from the group consisting of S, O, N, SO or SO₂; and
X is NH or CH₂ or NW;
W is H or a C₁-C₃-alkyl group;
n is 0 or 1;
Y is CH₂ or -CH(CH₃)-;

R' is an optionally substituted C₁-C₂₀-alkyl group, wherein one or two non-consecutive methylene groups may independently be replaced by O or S and the substituents may be as defined above for formulae (I)A and (II)A.

Preferred substituents for R' are alkyl, cycloalkyl, -OR^{b}, -SR^{b}, -CO₂R^{b}, -CONWR^{b}, -SO₂R^{b}, - NWSO₂R^{b}, -NW₂, fluoro, trifluoroalkyl, optionally substituted hydroxyalkyl, optionally substituted aminoalkyl, optionally substituted aryl, optionally substituted heteroaryl, *N-*methylpiperazinyl, *N*-morpholinyl, wherein R^{b} may independently be selected from H or C₁-C₅ alkyl

Compounds according to formula (III)A are preferred with R being an optionally substituted pyrrole, pyrazole, imidazole, imidazoline, imidazolidine, oxazole, oxazoline, oxazolidine, isoxazole, isoxazoline, isoxazolidine, thiazole, thiazoline, thiazolidine, isothiazole, isothiazoline, isothiazolidine, thiophen, furan, phenyl, cyclopentadien, hydroxyphenyl or piperidine.

Compounds according to formula (III)A are particularly preferred with R₁ and R together forming a substituted piperidine ring. In an even more preferred embodiment said piperidine ring is substituted by a group -C(O)NR'''₂, whereby R"' may independently be selected from H or C₁-C₅-alkyl, preferably ethyl. Regarding the stereochemistry of the bond between the pperidine and the -C(O)NR'''₂ group, both the (R) and (S) derivative are particularly preferred embodiments of the invention.

Compounds according to formula (III)A are especially preferred with R being an optionally substituted phenyl, or pyrazole.

Especially preferred are the aforementioned compounds according to formula (III)A in case of R being a pyrazole wherein the pyrazole is N-substituted, even more especially preferred by C₁-C₄-alkyl. Said pyrazole ring may preferably be substituted by a further group on one of its carbon atoms, more preferably by C₁-C₄-alkyl, even more preferably by a butyl group, most preferably by a t-butyl group.

Especially preferred are the aforementioned compounds according to formula (III)A in case of R being a phenyl wherein the phenyl is substituted by one or more F, Cl, Br or CF₃.

Further, compounds according to formula (III)A are preferred with R' being an optionally substituted C₉-C₂₀-alkyl group, wherein one or two non-consecutive methylene groups may independently be replaced by O or S and the substituents may be as defined above. More preferably, in the compounds according to formula (III)A R' is and optionally substituted C₉-C₂₀-alkyl group wherein the substituents may be as defined above. Compounds according to formula (I) are preferred with R being an optionally substituted C₁₀-C₁₂-alkyl group in which one carbon atom may be replaced by an oxygen atom, most preferred a C₁₀-alkyl group.

In yet another embodiment, the compositions of the present invention comprise, in part, compounds of Formula (III): 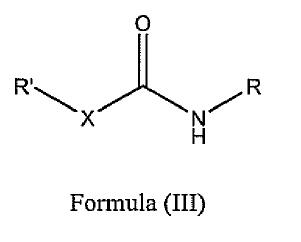 wherein R is a 5 or 6-membered monocyclic or polycyclic optionally substituted aromatic or non-aromatic ring system which may contain one or more group(s) X^{b}, wherein X^{b} is selected from the group consisting of S, O, N, SO or SO₂; and
X is C or N;
R' is an optionally substituted C₁-C₂₀-alkyl group.

The definitions and preferred embodiments as outlined for formula (III)A apply to Formula III as well.

Thus, in one embodiment subject of the present invention are compounds according to Formula (III) for the treatment of areas of wounds.

One embodiment of the invention are the compounds of formula (I)A-(III)A and the compounds of Formulas (I)-(III) for the treatment of an area of compromised skin integrity where bacterial pathogens may colonize resulting in infection. Such areas of compromised skin integrity include open wounds, lacerations, incisions, abrasions, puncture wounds, penetration wounds, skin tears, as well as chronic wound types such as pressure ulcers, venous and arterial ulcers and diabetic foot ulcers. The compositions of the present invention are especially useful for the treatment of human skin diseases caused by bacteria through the inhibition of the bacterial quorum sensing cascade rendering the pathogen avirulent. Such diseases include skin and soft tissue infections including wound infections caused by *Pseudomonas aeruginosa, Pseudomonas maltophilia, Enterobacter* species, *Klebsiella* species, *Serratia* species, *Escherichia coli, Proteus mirabilis, Morganella morganii, Providencia rettgeri, Proteus vulgaris, Providencia* species, *Citrobacter* species, *Acinetobacter calcoaceticus, Acinetobacter baumannii, Staphylococcus aureus, Staphylococcus epidermidis, Enterococcus* species, *Candida albicans, Fusarium sp., Corynebacterium diphtheriae, Clostridium difficile*, and *Clostridium perfringens*. Upon treatment of the skin, the inventive compositions remove, diminish, detach or disperse a bacterial biofilm from a living surface such as skin. This method is also useful as a prophylactic measure to prevent biofilm formation on the living surface by treating the surface with the present compositions before bacterial colonization can initialize.

In one embodiment, the compounds of formula (I)A-(III)A and the compounds of Formulas (I)-(III) can be also used in their corresponding salts form with inorganic or organic acids or bases. Examples of such salts are, e.g., alkali metal salts, in particular sodium and potassium salts, hydrochloride or ammonium salts.

Examples of pharmaceutically acceptable salts comprise, without limitation, non-toxic inorganic or organic salts such as acetate derived from acetic acid, aconitate derived from aconitic acid, ascorbate derived from ascorbic acid, benzoate derived from benzoic acid, cinnamate derived from cinnamic acid, citrate derived from citric acid, embonate derived from embonic acid, enantate derived from heptanoic acid, formiate derived from formic acid, fumarate derived from fumaric acid, glutamate derived from glutamic acid, glycolate derived from glycolic acid, chloride derived from hydrochloric acid, bromide derived from hydrobromic acid, lactate derived from lactic acid, maleate derived from maleic acid, malonate derived from malonic acid, mandelate derived from mandelic acid, methanesulfonate derived from methanesulfonic acid, naphtaline-2-sulfonate derived from naphtaline-2-sulfonic acid, nitrate derived from nitric acid, perchlorate derived from perchloric acid, phosphate derived from phosphoric acid, phthalate derived from phthalic acid, salicylate derived from salicylic acid, sorbate derived from sorbic acid, stearate derived from stearic acid, succinate derived from succinic acid, sulphate derived from sulphuric acid, tartrate derived from tartaric acid, toluene-p-sulfate derived from p-toluene-sulfonic acid and others. Such salts can be produced by methods known to someone of skill in the art and described in the prior art.

Other salts like oxalate derived from oxalic acid, which is not considered as pharmaceutically acceptable can be appropriate as intermediates for the production of the compounds of formula (I)A-(III)A and compounds of the Formulas (I)-(III) or a pharmaceutically acceptable salt thereof or stereoisomer thereof.

In a preferred embodiment, the compositions of the present invention comprise a therapeutically effective amount of at least one bacteriostatic or antimicrobial compound or a combination thereof. Such a compound is preferably included in an amount of about 1% w/w to 99% w/w, more preferably from 10% w/w to 90% w/w, and most preferably from 30% w/w to 70% w/w based on the total weight of the composition or medicament. Examples of suitable compounds include benzoyl peroxide and clindamycin. In an alternative embodiment, the compositions comprise at least one topical antibacterial compound such as sulfacetamide, mupirocin, bacitracin, neomycin, gentamycin, erythromycin, polymyxin B sulphate, fucidic acid, chlorhexidine acetate, and chlorhexidine gluconate.

In a particularly preferable embodiment, silver-containing sulfa derivative compounds such as silver sulfadiazine which exhibit both bacteriostatic and antimicrobial properties are combined with at least one compound of formula (I)A-(III)A and (I)-(III). A sulfa derivative, if included, is preferably included in an amount from about 1% w/w to 99% w/w, more preferably from 10% w/w to 90% w/w, and most preferably from 30% w/w to 70% w/w, all weights based on the total weight of the composition. This combination produces a synergistic reduction of both planktonic bacteria and biofilms and also aids in the prevention of further biofilm formation as demonstrated by the Examples described herein. For example, it is possible to reduce planktonic bacteria by up to 90% or even up to 99% by use of the bacteriostatic/antimicrobial, and to drastically reduce, or even completely eliminate the formation ofbiofilms up to an amount of 95% or even 99% by use of a compound of Formula I, II and/or III. The sulfadiazine moiety is believed to provide bacteriostatic action against sensitive organisms and, when combined with silver, may result in a bacteriocidal composition. The mechanism of silver sulfadiazine's antibacterial action has not been fully elucidated by medical research; however exposure to the drug is believed to result in structural changes in the bacterial cell membrane which includes distortion and enlargement of the cell and a weakening of the cell wall membrane. The silver sulfadiazine molecule dissociates and the silver moiety is bound to the bacterial cells. After penetrating the cell wall, the silver moiety is believed to attach to the bacterial deoxyribonucleic acid (DNA) and prevent bacterial cell proliferation.

In one embodiment, pharmaceutical compositions are prepared that comprise, in addition to the aforementioned compositions, additives or adjuvants commonly used in galenic formulations, such as, e.g., fillers, extenders, disintegrants, binders, glidants, wetting agents, stabilizers, emulsifiers, preservatives, sweetening agents, colorants, flavorings or aromatizers, buffer substances, and furthermore solvents or solubilizers or agents for achieving a depot effect, as well as salts for modifying the osmotic pressure, coating agents or antioxidants. Such pharmaceutical compositions can also contain two or more compounds of the Formulas (I)A-(III)A and (I)-(III) or their pharmacologically acceptable salts and also other therapeutically active substances.

In one embodiment, the compositions of the present invention can be used variety of formulations including creams, lotions, solutions, powders or as a coating or incorporated into a dressing. The present compositions are preferably applied to the surface of an area of compromised skin integrity as an ointment comprising the present compositions, alone or together with other materials such as conventional surfactants, preferably sodium dodecyl sulfate, or detergents, biocides, fungicides, antibiotics, pH regulators, perfumes, dyes or colorants and other customary, usually inert carrier materials or excipients.

To prepare a pharmaceutical preparations or medicament in an ointment formulation, pharmaceutically inert inorganic or organic excipients can be used. Specifically, DMSO, propylene glycol, PEG 400, PEG 1500, and PEG 4000 maybe combined in varying amounts depending on the corresponding amount of the active ingredients such as a compound of Formulas I-III, a bacteriostatic compound, an antimicrobial compound or any combination thereof. Preferably, the amounts range from about 1 to 50grams of propylene glycol, about 1 to 50 grams of PEG 400, about 1 to 50 grams of PEG 1500, and about 1 to 50 grams of PEG 4000. More preferably, the amounts range from about 10to 30 grams of propylene glycol, about 10 to 30 grams of PEG 400, about 5 to 25 grams of PEG 1500, and about 5 to 25 grams of PEG 4000. In a particularly advantageous embodiment, the amounts range from about 13.06 grams of propylene glycol, about 20.90 grams of PEG 400, about 9.14 grams of PEG 1500, and about 9.14 grams of PEG 4000. Each of these components are melted in a mixture on a water bath at about 35°C to 85°C. More preferably, these components are melted in a mixture on a water bath at about 45°C to 75°C. In a particularly advantegous embodiment, these components are melted in a mixture on a water bath at about 60°C. About 1 to 5 ml of DMSO is added to the clear melted mixture. More preferably, about 2 to 4 ml are added while in a particularly advantageous embodiment, about 2.75 ml of DMSO are added to the clear melted mixture.

The mixture is then stirred preferably with a pestle until the mixture is cooled to room temperature resulting in a 50 mg white, semi-solid ointment. The active ingredients are added in amounts depending on the dosage. The appropriate dosage will vary depending on the mode of administration, the particular condition to be treated and the effect desired. Suitable amounts to be administered to mammalian in particular humans preferably range from about 1 to 5000 mg. More preferably, the amounts range from 5 to 1000 mg. Most preferably, the amounts range from 10 to 500 mg. The compounds of Formula I, II and III of the present invention can be used alone, in combination with other compounds or in combination with other active compounds, for example with active ingredients already known for the treatment of the afore mentioned diseases, whereby in the latter case a favorable synergistic effect is noticed.

In one embodiment, the compositions are useful for the treatment of an area of compromised skin integrity where bacterial pathogens may colonize resulting in infection. Such areas of compromised skin integrity include open wounds, lacerations, incisions, abrasions, puncture wounds, penetration wounds, skin tears, as well as chronic wound types such as pressure ulcers, venous and arterial ulcers and diabetic foot ulcers. The compositions of the present invention are especially useful for the treatment of human skin diseases caused by bacteria through the inhibition of the bacterial quorum sensing cascade rendering the pathogen avirulent. Such diseases include skin and soft tissue infections including wound infections caused by *Pseudomonas aeruginosa, Pseudomonas maltophilia, Enterobacter* species, *Klebsiella* species, *Serratia* species, *Escherichia coli, Proteus mirabilis, Morganella morganii, Providencia rettgeri, Proteus vulgaris, Providencia* species, *Citrobacter* species, *Acinetobacter calcoaceticus, Acinetobacter baumannii, Staphylococcus aureus, Staphylococcus epidermidis, Enterococcus* species, *Candida albicans, Fusarium sp., Corynebacterium diphtheria, Clostridium difficile,* and *Clostridium perfringens.* Upon treatment of the skin, the inventive compositions remove, diminish, detach or disperse a bacterial biofilm from a living surface such as skin. This method is also useful as a prophylactic measure to prevent biofilm formation on the living surface by treating the surface with the present compositions before bacterial colonization can initialize.

Advantages of the present invention will be apparent from the description of the examples that follow. The following examples exhibit merely a preferred embodiment of the present invention.

### EXAMPLES

The effect of three quorum sensing blocking compositions on *Pseudomonas aeruginosa* infected bums was determined using a porcine model. Two swine were used as experimental research animals since swine skin is morphologically similar to human skin. The young female specific pathogen free (SPF) pigs weighing 25-30 kg were kept in-house for two weeks prior to initiating the experiment. The animals were fed a basal diet *ad libitum* and housed individually in an American Association for Accreditation of Laboratory Animal accredited animal facility with controlled temperature (19-21°C) and lights (12h/12h LD).

Each animal was anesthetized with Telazol HCl (5 mg/kg), Xylazine (0.2 mg/kg), Atropine (0.05 mg/kg) I.M. and inhalation of an isofluorane and oxygen combination. Hair on the back of the pig was clipped with standard animal clippers. Skin on the back and sides surrounding and including the wound area of the animals was prepared by washing with a non-antibiotic soap (Neutrogena^{®}) and sterile water. The animals were blotted dry with sterile gauze. Forty-eight second degree burn wounds were made on the paravertebral and thoracic area by using five specially designed cylindrical brass rods weighing 358g each that were heated in a boiling water bath to 100°C (See Davis SC, Mertz PM and Eaglstein WH. Second Degree Burn Healing: The Effect of Occlusive Dressings and a Cream. J. Surg. Res. 48:245-248, 1990; see also Mertz PM, Davis SC, Franzén L, Uchima FD, Pickett MP, Pierschbacher MD, Polarek JW: Effect of an RGD Peptide-Containing Artificial Matrix on Epithelial Sheet Migration and Experimental Second-Degree Burn Wound Healing. J Burn Care and Rehabilitation 17(3): 199-206, 1996). A rod was removed from the water bath and wiped dry before it was applied to the skin surface to prevent water droplets from creating a steam burn on the skin. The brass rod was held at a vertical position on the skin (six seconds), with all pressure supplied by gravity, to make a burn wound 8.5 mm diameter x 0.8 mm deep.

Immediately after burning, the roof of the burn blister was removed with a sterile spatula. The burn wounds were then treated or inoculated with a fresh culture of a pathogenic wound isolate *Pseudomonas aeruginosa* (American Type Culture Collection (ATCC) 27312). The bacterial culture was recovered per ATCC standard recovering protocol. The challenge inoculum suspensions were prepared by scraping the overnight growth from a culture plate into 5 mL of normal saline. This resulted in a suspension concentration of approximately 10⁸ colony forming units/ml (CFU/ml) of bacteria. Serial dilutions were made until a concentration of 10⁶ cfu/mL was achieved. The inoculum was vortexed and 25 µL of the suspension used to inoculate each wound. Serial dilutions of the suspension were plated onto culture media to quantify the exact concentration of viable organisms used for this experiment. After inoculation some of the wounds were covered with a polyurethane dressing for 48 hours, which allowed the bacteria to colonize the wound and develop a biofilm.

Four (4) wounds were cultured at 48 hours post treatment. A sterile steel or glass cylinder (22mm outside diameter) was placed over the wound area. One (1) ml of neutralizing scrub solution was pipetted into the cylinder and the site gently washed three times to remove the loosely attached bacteria. This aliquot was aspirated and saved for assessment of viable bacteria. This aliquot, referred to herein as the flush culture, represents the planktonic bacteria. The same wound was then encircled using another sterile cylinder. One (1) ml of scrub solution was deposited and the wound scrubbed with a sterile Teflon spatula for 30 seconds to remove the attached bacteria. This aliquot, referred to herein as the scrub culture, represented biofilm bacteria.

Serial dilutions were made of both flush and scrub culture samples and the extent of microbiological contamination was assessed using the Spiral Plater System (Spiral Biotech, Norwood, MA) that deposited a 50-µl aliquot of the flush or scrub bacterial suspension over the surface of a rotating agar plate. *Pseudomonas* agar was used to isolate the initial inoculum. All plates were incubated aerobically overnight (∼18 hours) at 37°C, after which the number of colonies were counted.

On day one (24 hours post treatment), there was slight erythema but no signs of infections observed in either pig on the Planktonic or Biofilm side (all treatments). Some areas of the normal skin had a very thin residue post application (all groups). The test articles appeared to have dissolved completely into the wound and normal skin.

On day two (48 hours post treatment), wounds in the biofilm side developed slightly more erythema but no infections. On day 3 (72 hrs post treatment) most of the erythema disappeared from wounds (all treatment groups). A few pustules developed (normal skin) in group D (vehicle) in both pigs. On day 4 (96 hours post treatment), all erythema disappeared completely on both sides and both pigs except for a few of the wounds in the Biofilm side of one pig. The pustules persisted during day 4 in both pigs. Pustules were cultured and grown on Mannitol Salt media indicating the presence of *Staphylococcus aureus*, a normal constituent of the skin flora. After the colonies were counted, the colony forming units per mL (CFU/ml) were calculated.

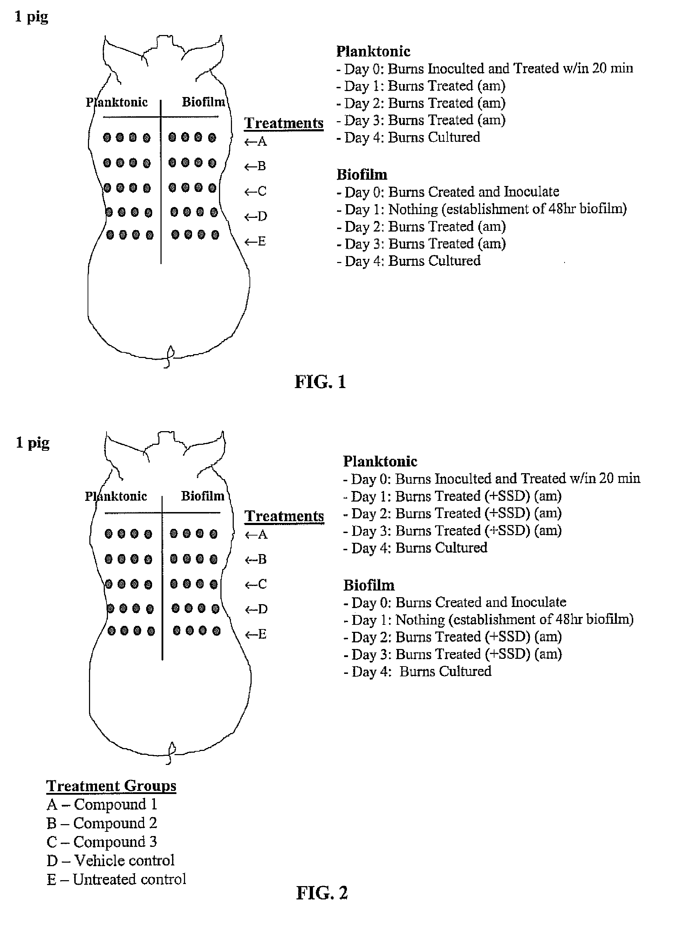

Wounds were treated with ointments containing quorum-sensing blocking compounds and individually covered with Tegaderm polyurethane film dressings. As illustrated in Figure 1, the first pig was treated with Compounds 1-3 illustrated in Table 1 without silver sulfadiazine. As illustrated in Figure 2, the second pig was treated with an inventive solution containing quorum-sensing blocking Compounds 1-3 combined with equal parts of silver sulfadiazine (SSD, Tyco Health Care/Kendall, Mansfield, MA, U.S.A). Coban wrap was used to the secure dressings on the pig.

Treatment with the active agents in the absence of SSD resulted in a reduction in *P*. *aeruginosa* counts by comparison to the untreated controls. The vehicle control had no effect on *P. aeruginosa* as compared to the untreated control. Compound 1 had the largest reduction of counts of planktonic *P. aeruginosa* when used within 20 minutes of wound inoculation or on established biofilms. Compound 2 and Compound 3 also resulted in lower counts of planktonic *P. aeruginosa* when applied within 20 minutes of inoculation.

Using the same treatment regimen, Compound 1 resulted in a 1 Log reduction of biofilm *P*. *aeruginosa* when used within 20 minutes. Treatment of wounds within 20 minutes of inoculation with Compound 1 (+SSD) and Compound 2 (+SSD) resulted in a 3 Log reduction of *P. aeruginosa* when compared to the vehicle control. Treatment with Compound 3 (+SSD) resulted in a 1.5 Log reduction of planktonic *P. aeruginosa* when compared to the vehicle (+SSD).

Treatment of established biofilms indicated that treatment with Compound 3 (+SSD) combination resulted in a noticeable reduction of planktonic *P. aeruginosa* compared to the vehicle. Vehicle (+SSD) resulted in reduction of planktonic *P. aeruginosa* in all instances when compared to the untreated control.

Treatment of wounds within 20 minutes of inoculation with Compound 1 and Compound 2 resulted in a 2 Log and 1.5 Log reduction of biofilm bacteria when compared to the vehicle control, respectively. Treatment with Compound 3 (+SSD) combination resulted in a 1 Log reduction of biofilm *P. aeruginosa* when compared to the vehicle.

Treatment of established biofilms indicated that treatment with Compound 3 (+SSD) and Compound 1 (+SSD) resulted in a 0.5 Log reduction of biofilm bacteria compared to the vehicle. Vehicle (+SSD) resulted in reduction of biofilm bacteria in all instances when compared to the untreated control.

Thus, as demonstrated herein, the compositions containing least one quorum sensing blocking compound alone (i.e., no SSD) have been shown to reduce counts of both planktonic bacteria and biofilm formations. In addition, the compositions containing silver sulfadiazine have demonstrated an unexpected ability to synergistically reduce both planktonic bacteria and biofilm formations as well as the ability to aid in the prevention of further biofilm formation.

**Table 1: List of quorum sensing blocking compounds.**

| No. | Compound |
|---|---|
| 1 | 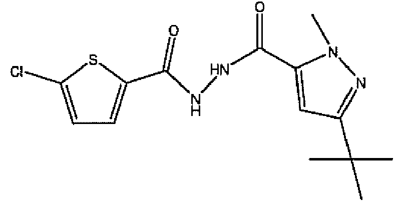 |
| 2 | 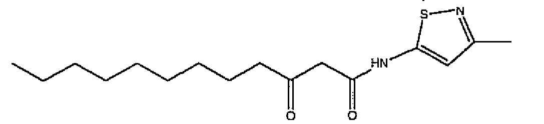 |
| 3 | 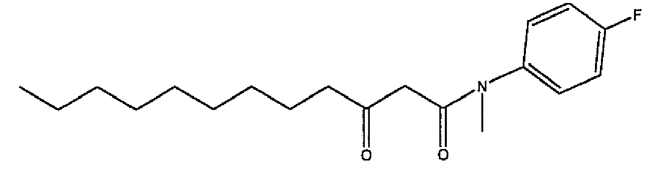 |
| 4 | 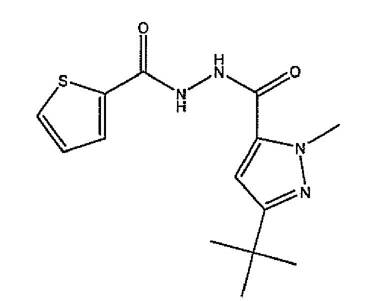 |
| 5 | 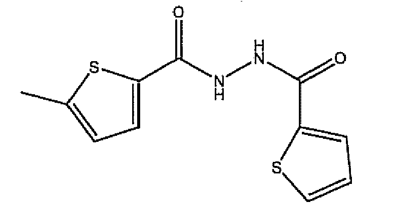 |
| 6 | 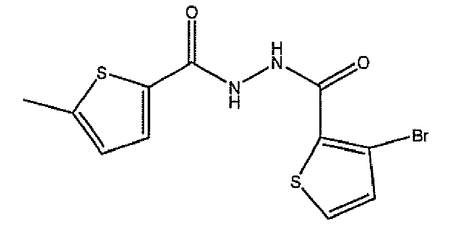 |
| 7 | 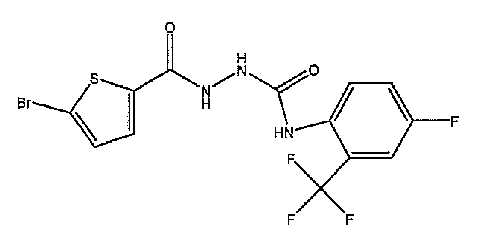 |
| 8 | 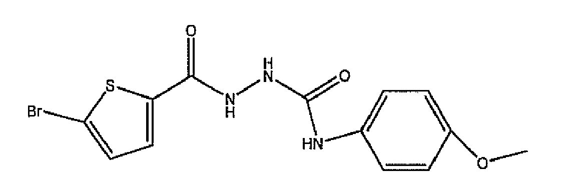 |
| 9 | 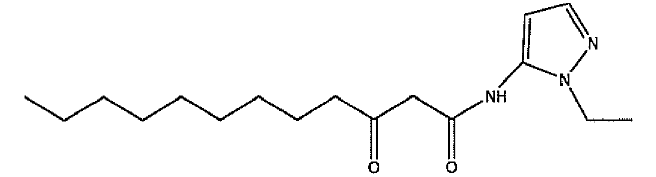 |
| 10 | 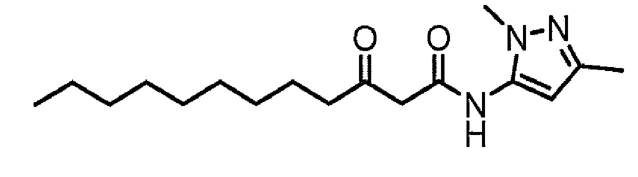 |
| 11 | 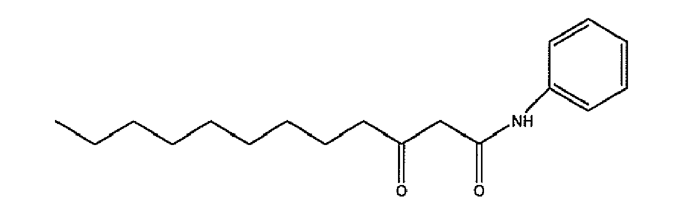 |
| 12 | 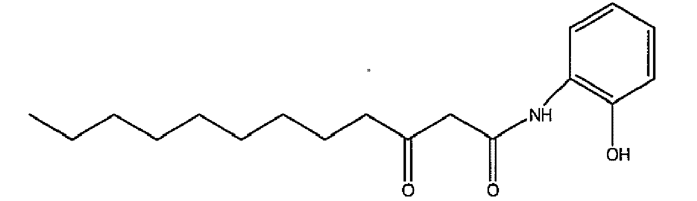 |
| 13 | 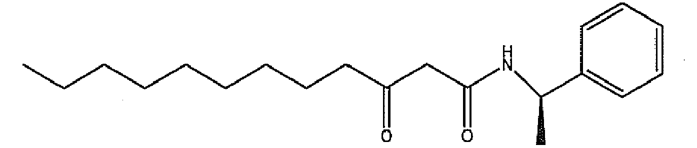 |
| 14 | 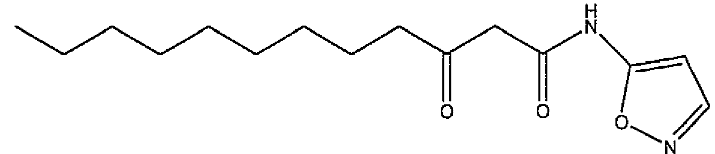 |
| 15 | 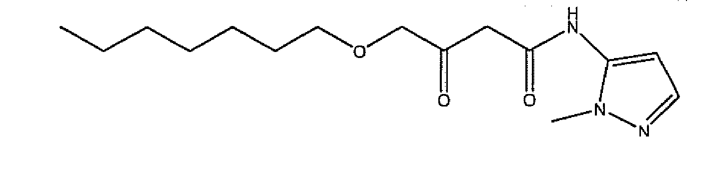 |
| 16 | 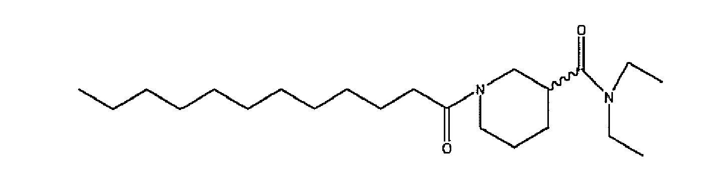 |
| 17 | 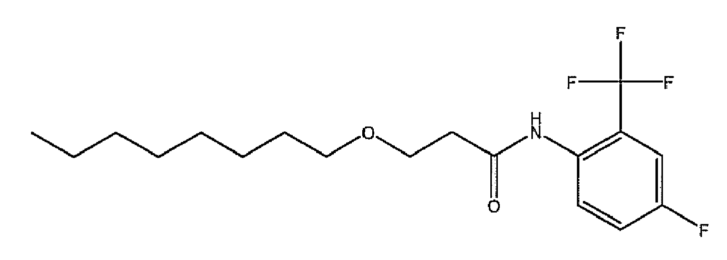 |
| 18 | 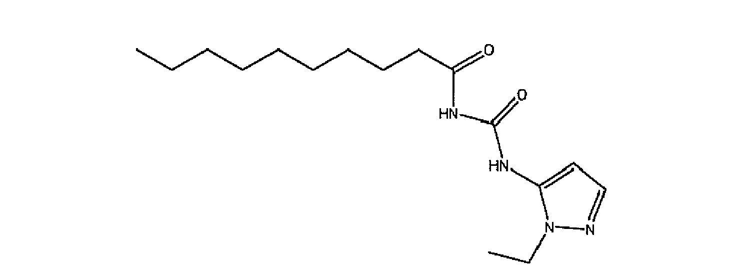 |
| 19 | 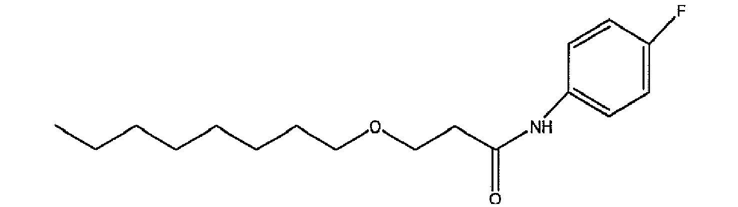 |
| 20 | 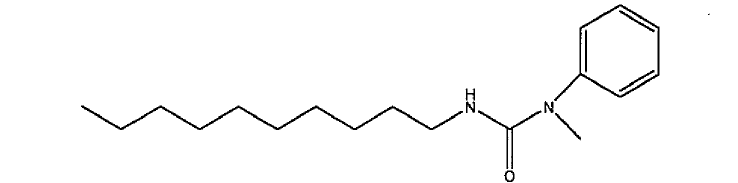 |
| 21 | 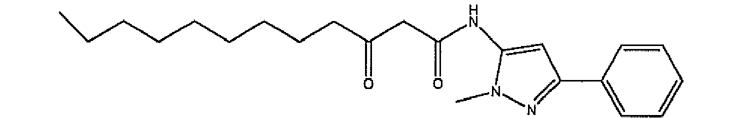 |
| 22 | 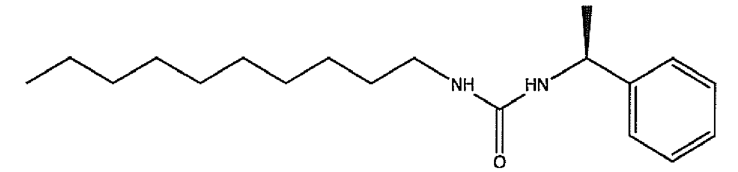 |
| 23 | 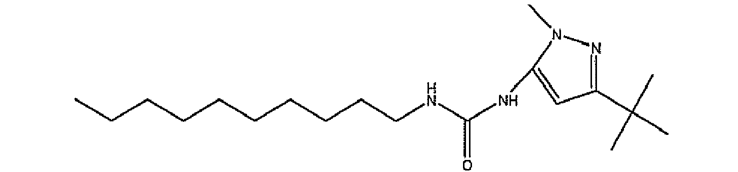 |
| 24 | 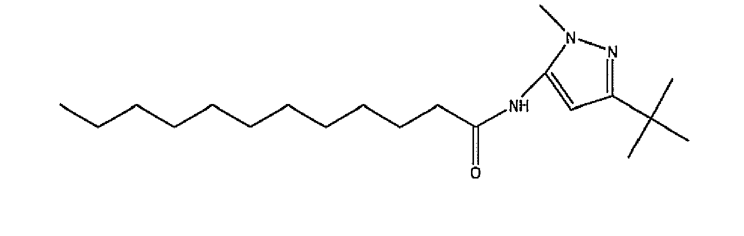 |

## Claims

1. A compound according to Formula (I)A for the treatment of areas of wounds: 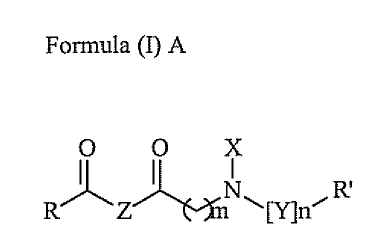 wherein R is an optionally substituted C₁-C₂₀-alkyl group, wherein one or two non-consecutive methylene groups may independently be replaced by O or S;
X is H or a C₁-C₃-alkyl group;
Z is NH or CH₂ or NX;
R' is an optionally substituted aromatic or non-aromatic ring system consisting of one or two 5- or 6-membered rings wherein in each ring one or more of the carbon atoms may be replaced by a group X^{b}, wherein X^{b} is selected from the group consisting of S, O, N, SO or SO₂;
the substituted ring system carries one or more substituents R" on the carbon or hetero atoms of said ring system;
R" is independently H, OH, SH, O-alkyl, hydroxyalkyl, hydroxyalkylamine, cycloalkyl, halogen, haloalkyl, haloalkyloxy, NO₂, CN, alkyl, aryl or heteroaryl;
Y is an optionally substituted C₁-C₂₀-alkyl group;
n and m are independently 0 or 1.

2. A compound according to claim 1 for the treatment of areas of wounds,
wherein R' is an optionally substituted isothiazole, phenyl, pyrazole, cyclopentadiene, hydroxyphenyl, or isoxazole.

3. A compound according to Formula (II)A for the treatment of areas of wounds: 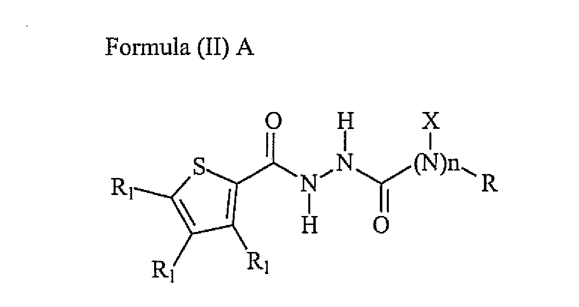 wherein R is an optionally substituted aromatic or non-aromatic ring system consisting of one or two 5- or 6-membered rings wherein in each ring one or more of the carbon atoms may be replaced by a group X^{b}, wherein X^{b} is selected from the group consisting of S, O, N, SO or SO₂; and
wherein R₁ is interpedently of each other H, halogen, NO₂, CN, alkyl, OH, cycloalkyl, SH, alkylthio, hydroxyalkylamino, hydroxyalky, aryl or heteroaryl,
wherein X is H or a C₁-C₃-alkyl group;
n is 0 or 1.

4. A compound according to claim 3 for the treatment of areas of wounds,
wherein R is an optionally substituted pyrazole, thiophen, or phenyl.

5. A compound according to Formula (III)A for the treatment of areas of wounds: 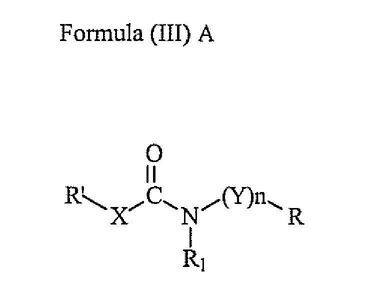 wherein R is an optionally substituted aromatic or non-aromatic ring system consisting of one or two 5- or 6-membered rings wherein in each ring one or more of the carbon atoms may be replaced by a group X^{b}, wherein X^{b} is selected from the group consisting of S, O, N, SO or SO₂ and
R₁ is H or a C₁-C₃-alkyl group, or
R₁ and R taken together may form a 5 or 6-membered optionally substituted aromatic or non-aromatic ring system wherein one or more of the carbon atoms may be replaced by a group X^{b}, wherein X^{b} is selected from the group consisting of S, O, N, SO or SO₂; and
X is NH or CH₂ or NW;
W is H or a C₁-C₃-alkyl group;
n is 0 or 1;
Y is CH₂ or -CH(CH₃)-;
R' is an optionally substituted C₁-C₂₀-alkyl group, wherein one or two non-consecutive methylene groups may independently be replaced by O or S.

6. A compound according to claim 5, wherein R₁ and R form together a ring that is a substituted piperidine ring, wherein the piperidine is substituted by a group -C(O)N(Et)₂.

7. Compounds according to to claim 5, wherein R is a optionally substituted phenyl, or pyrazole.

8. A compound selected from the group comprising the following Fomulae for the treatment of areas of wounds:

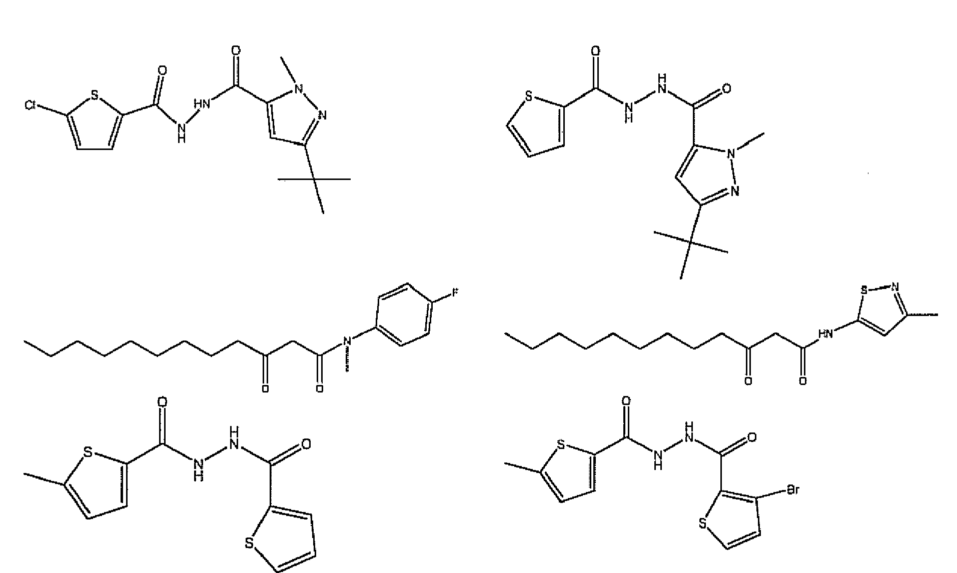

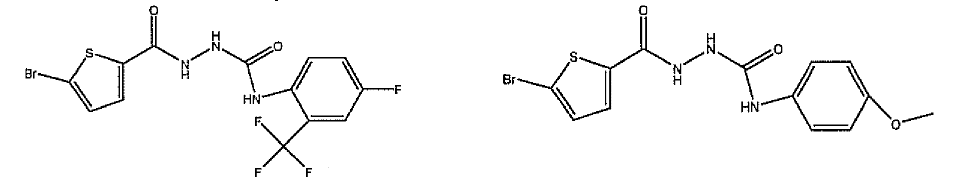

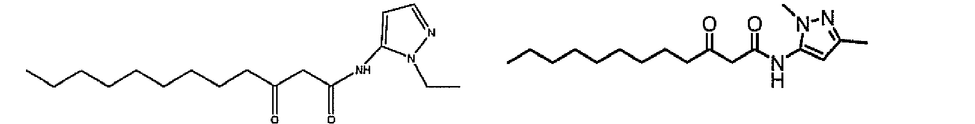

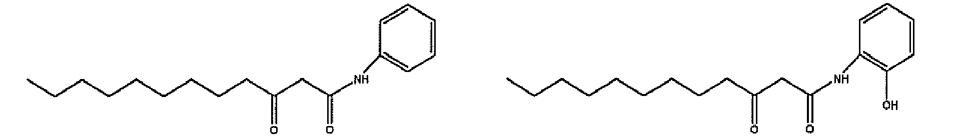

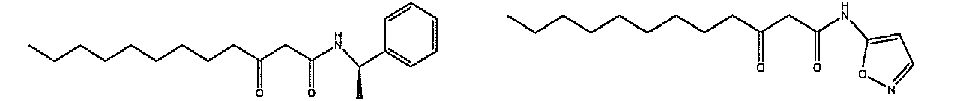

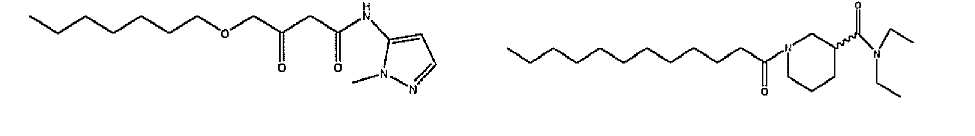

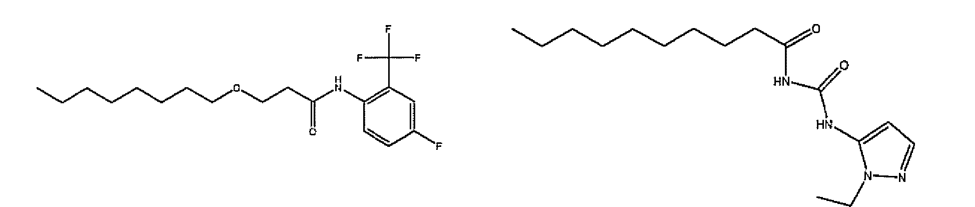

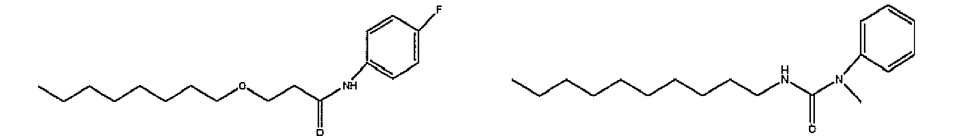

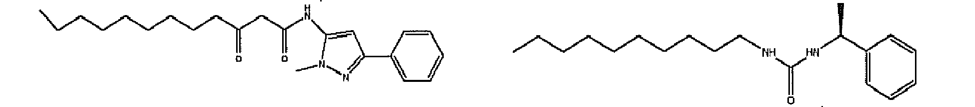

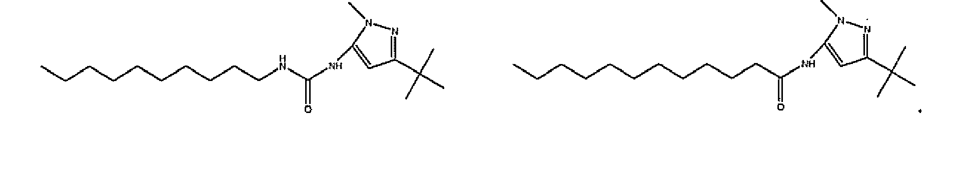

9. A pharmaceutical composition comprising one or more compounds according to any of claims 1 to 8.

10. A Composition according to claim 9, additionally comprising a therapeutically effective amount of at least one compound capable of either preventing infection by inhibiting growth or action of a least one bacterium or capable of destroying organisms that might carry a disease.

11. The use of the compounds and compositions as defined in any one of Claims 1-10 for inhibiting quorum sensing of the microorganism.

12. A compound according to formulas (I)A, IIA or_III(A) for treating or preventing bacterial infection on or around an area of compromised skin integrity,..

13. A compound according to claim12, wherein the bacterial infection comprises a skin and/or soft tissue infection caused by a microrgansim selected from the group consisting of *Pseudomonas aeruginosa, Pseudomonas maltophilia, Enterobacter* species, *Klebsiella* species, *Serratia* species, *Escherichia coli, Proteus mirabilis, Morganella morganii, Providencia rettgeri, Proteus vulgaris, Providencia* species, *Citrobacter* species, *Acinetobacter calcoaceticus, Acinetobacter baumannii, Staphylococcus aureus, Staphylococcus epidermidis, Enterococcus* species, *Candida albicans, Fusarium sp., Corynebacterium diphtheriae, Clostridium difficile*, and *Clostridium perfringens*.

14. A compound according to formulas (I)A, IIA or_III(A) for treating biofilm and/or inhibiting of the formation of biofilm on living membrane tissue, wherein the biofilm comprises at least a bacterium having a quorum sensing system, and the method further comprises regulating the quorum sensing system of the bacterium.
